# EUROPEAN PATENT APPLICATION

(11) **EP 2 130 911 A1**
(43) Date of publication of application: **09.12.2009**
(21) Application number: 09012264.9
(22) Date of filing: 17.01.2007
(51) Int. Cl.: C12N 5/06, A61F 2/14, A61L 27/00

(54) **Feeder cell derived from tissue stem cell**

(30) Priority: 08.02.2006 JP 2006030997
(62) Divisional of application: 07706922.7
(71) Applicant: Keio University, Tokyo 108-8345 (JP)
(72) Inventor: Shimmura, Shigeto, Tokyo 160-8582 (JP); Miyashita, Hideyuki, Tokyo 160-8582 (JP); Yoshida, Satoru, Tokyo 160-8582 (JP); Tsubota, Kazuo, Tokyo 160-8582 (JP)
(74) Representative: Ford, Hazel

(57) **Abstract**

It is an object of the present invention to provide a feeder cell with less variation in quality. The present invention relates to a feeder cell derived from a tissue stem and/or progenitor cell. A method of preparation of the feeder cell, a method of preparation of a cultured cell using the feeder cell, and a cell culturing kit are also provided.

## Description

### TECHNICAL FIELD

The present invention relates to a tissue stem cell-derived feeder cell and use thereof

### BACKGROUND ART

Transplantation of cultured epithelial sheets is used to treat stem cell exhaustion in the corneal limbus. Stem cell exhaustion in the corneal limbus is a condition where stem cells in the corneal epithelium are dead because of alkali injury or the like, leading to severe damage in eye sight as a result of invasion of opaque conjunctive (Non-Patent Document 1).

There are two types of methods for culturing epithelial cells. One type does not use feeder cells and the other type uses feeder cells. The latter type (using feeder cells) is superior in epithelial cell maintaining capacity (Non-Patent Document 2). Feeder cells are fibroblasts whose growth has been terminated, for example, by treatment with drugs. Feeder cells have functions of promoting the growth of epithelial cells and inhibiting the growth of fibroblasts mixed in (Non-Patent Document 3). Epithelial sheets cultured with 3T3 cells as a feeder layer have been used in clinical scenes for more than 20 years.

However, since 3T3 cells are derived from mouse, the risk of xenogeneic injection can not be eliminated completely. Transplantation of epithelial sheets cultured with 3T3 cells is classified as xenotransplantation in the guidelines provided by Ministry of Health, Labour and Welfare (Non-Patent Document 4).

In order to avoid this problem, human cells may be used as a feeder layer. Actually, it has been reported that epidermal cells (skin epithelial cells) can be cultured and maintained using human fibroblasts as a feeder layer (Non-Patent Document 5).

However, unlike 3T3 which is an established cell strain (i.e. immortalized cells) from mouse, normal human fibroblasts are known to get gradually aged through repeated subculture. This means that it is impossible to use cells of one lot permanently. Thus, the quality of feeder layers may vary because of subculture or difference in lot. It is predicted that such possibility will give adverse effect on the control of the quality of cultured epithelial sheets.

Recently, a method of culturing and maintaining brain or skin stem cells has been developed. Stem cells cultured by this method do not differentiate into epithelium, but they are capable of differentiating into fibroblasts. It has been reported that not only mouse cells but also human skin stem cells can be maintained for a long time by using this method (Non-Patent Document 6). The group of the present inventors has also succeeded in isolating and culturing stem cells from mouse corneal stroma using this method (Non-Patent Document 7).
Non-Patent Document 1: Ann Acad Med Singapore 2004; 33: 576-80
Non-Patent Document 2: CANCER RESEARCH 63, 7815-7824, November 15, 2003
Non-Patent Document 3: Proc. Natl. Acad. Sci. USA Vol. 76, No. 11, pp. 5665-5668, November 1979
Non-Patent Document 4: Guidelines on Epithelial System Regenerative Medicine Using 3T3J2 or 3T3NIH as Feeder Cells based on "Public Health Guidelines on Infectious Disease Issued in Xenotransplantaion", Hiroshi Yoshikura (Director, the National Institute of Infectious Diseases), Chief Researcher, Health Sciences Special Research Program funded by 2003 Health Labour Sciences Research Grant
Non-Patent Document 5: STEM CELLS 2003;21:481-494
Non-Patent Document 6: STEM CELLS 2005;23:727-737
Non-Patent Document 7: Investigative Ophthalmology & Visual Science, May 2005, Vol. 46, No.5, 1653-1658

### DISCLOSURE OF THE INVENTION

### PROBLEM FOR SOLUTION BY THE INVENTION

It is an object of the present invention to provide feeder cells with less variation in quality.

### MEANS TO SOLVE THE PROBLEM

If it is possible to use stem cells with the ability of self-replication (i.e., without variation in quality) as a source of feeder cells, it is expected that, theoretically, high quality cells of one lot can be used unlimitedly. Then, the present inventors have succeeded in culturing transplantable human epithelial sheets by using feeder cells prepared from mouse corneal stroma stem cells. Thus, the present invention has been achieved.

The present invention relates to the following inventions.
(1) A feeder cell derived from a tissue stem and/or progenitor cell.
(2) The feeder cell of (1) above, wherein the tissue stem and/or progenitor cell is derived from a mammal.
(3) The feeder cell of (2) above, wherein the mammal is human.
(4) The feeder cell of any one of (1) to (3) above, wherein the tissue stem and/or progenitor cell is derived from corneal stroma, skin or bone marrow mesenchyme.
(5) A method of preparing a feeder cell, comprising treating a tissue stem and/or progenitor cell to decrease the growth ability thereof, optionally after inducing the tissue stem and/or progenitor cell into a fibroblast.
(6) The method of (5) above, wherein the tissue stem and/or progenitor cell is derived from a mammal.
(7) The method of (6) above, wherein the mammal is human.
(8) The method of any one of (5) to (7) above, wherein the tissue stem and/or progenitor cell is derived from corneal stroma, skin or bone marrow mesenchyme.
(9) A method of preparing a cultured cell, comprising co-culturing a cell with the feeder cell of any one of (1) to (4) above.
(10) The method of (9) above, wherein the cell co-cultured with the feeder cell of any one of (1) to (4) above is derived from a mammal.
(11) The method of (10) above, wherein the mammal is human.
(12) The method of any one of (9) to (11) above, wherein the cell co-cultured with the feeder cell of any one of (1) to (4) above is selected from the group consisting of corneal cells, skin cells, gingival cells, cardiac cells, gastrointestinal cells, bone marrow cells, embryonic stem cells and oral mucosal cells.
(13) The method of (12) above, wherein the cell co-cultured with the feeder cell of any one of (1) to (4) above is a corneal epithelial cell or skin cell.
(14) The method of (13) above, wherein the cultured cell is stratified.
(15) The method of any one of (9) to (14) above, wherein the cultured cell is used for transplantation.
(16) A cell culturing kit comprising the feeder cell of any one of (1) to (4) above.

### EFFECT OF THE INVENTION

The advantage of the present invention resides in that homogeneous feeder cells can be supplied stably because stem cells are used as a cell source. Further, when a human cell is used as a feeder cell, it is possible to reduce the risk of xenogeneic infection.

The present specification encompasses the contents described in the specification and/or drawings of Japanese Patent Application No. 2006-030997 based on which the present application claims priority.

### BRIEF DESCRIPTION OF THE DRAWING

Fig. 1
   A: mouse stromal stem cells in culture; B: feeder cells obtained from the cells shown in A by treatment with drugs; C: practice of epithelial culture; and D: epithelial cells in culture.

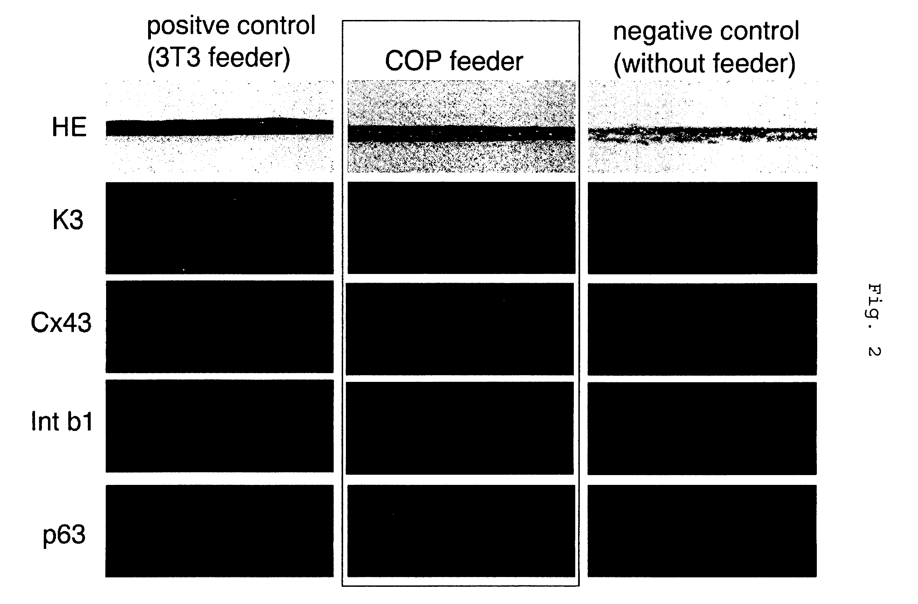

Fig. 2 shows tissue samples of cultured epithelial cells. HE: hematoxylin-eosin staining images; K3: immunostaining images of keratin-3 which is a corneal epithelial differentiation marker; Cx43: immunostaining images of connexin 43 which is an epithelial cell differentiation marker; Int b1: immunostaining images of integrin β1 which is an indicator for undifferentiated epithelium; and p63: immunostaining images of p63 which is a marker for undifferentiated epithelial cells.

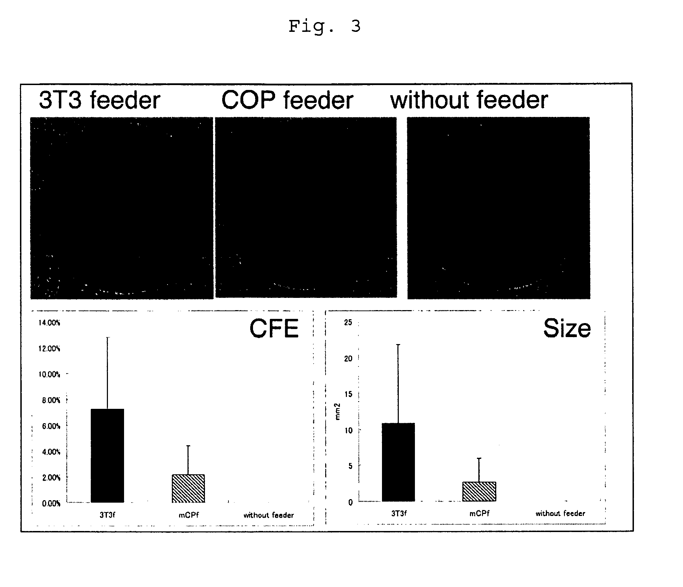

Fig. 3 shows rhodamine B staining images of 1000 epithelial cells after co-culturing with individual feeder cells for two weeks. Left lower panel: epithelial colony forming efficiency (CFE). Right lower panel: average size of epithelial colonies.

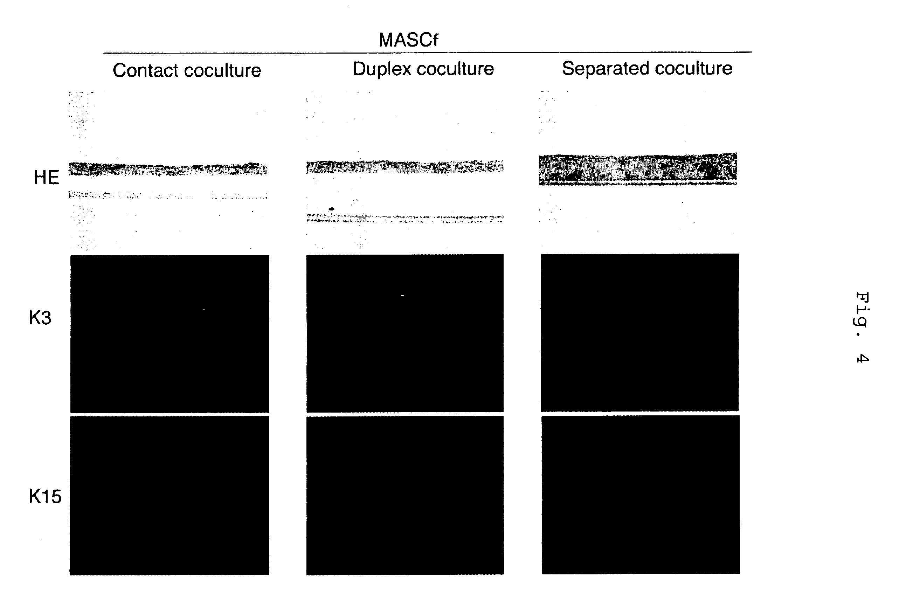

Fig. 4 shows cultured epithelial sheets prepared with epithelial cells and human bone marrow mesenchymal stem cell-derived feeder cells (MASCf). Contact co-culture: epithelial sheets cultured in contact with feeder cells by seeding epithelial cells together with feeder cells in culture inserts. Duplex co-culture: epithelial sheets cultured in contact with feeder cells and under continuous supply of humoral factors by seeding feeder cells both in culture inserts and wells beneath the inserts. Separate co-culture: epithelial sheets co-cultured with feeder cells without contact by seeding feeder cells in only the wells beneath culture inserts. HE: hematoxylin-eosin staining images; K3: immunostaining images of keratin-3 which is a corneal epithelial differentiation marker; and K15: immunostaining images of keratin-15 which is a marker for corneal limbal epithelium.

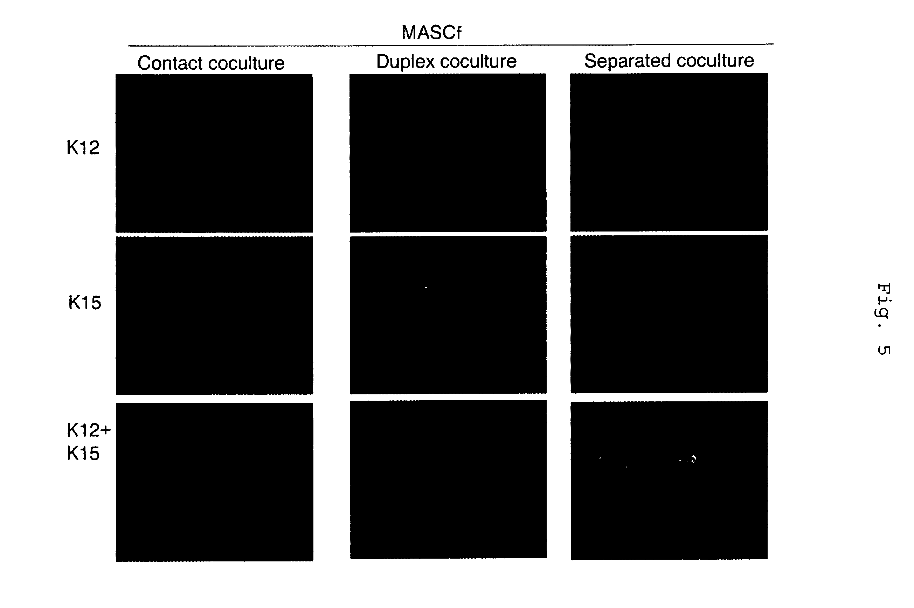

Fig. 5 shows cultured epithelial sheets prepared with epithelial cells and human bone marrow mesenchymal stem cell-derived feeder cells (MASCf). K12: immunostaining images of keratin-12 which is a corneal epithelial differentiation marker; K15: immunostaining images of keratin-15 which is a marker for corneal limbal epithelium; and K12+K15: keratin-12 and keratin-15-double staining images.

### BEST MODE FOR CARRYING OUT THE INVENTION

Hereinbelow, the present invention will be described in details.

The present invention provides a feeder cell derived from a tissue stem and/or progenitor cell.

The tissue stem and/or progenitor cell may be derived from a mammal (e.g., human or mouse). Preferably, the cell is derived from a mammal, especially, human.

The tissue stem and/or progenitor cell may be derived from either adults or embryos.

The tissue stem and/or progenitor cell may be derived from any tissue, for example, bone marrow, muscle, nerve, skin, cornea, liver, spleen, small intestine, oral mucosa or fat. Preferably, the cell is derived from cornea (especially, corneal stroma), skin or bone marrow (especially, bone marrow mesenchyme).

The tissue stem and/or progenitor cell may be prepared as described below. Briefly, a tissue section of interest is collected and unnecessary portions are removed as much as possible. After an overnight treatment with disperse at 4°C, epithelium is removed. Then, the tissue is melted by collagenase treatment for 1 to 2 hours at 37°C. Cells are harvested by centrifugation and cultured in a serum-free medium for culturing tissue stem and/or progenitor cells. The cells are subcultured every 2 to 3 weeks.

The feeder cell of the present invention may be prepared by treating a tissue stem and/or progenitor cell to decrease the growth ability thereof, optionally after inducing the tissue stem and/or progenitor cell into a fibroblast.

The induction of a tissue stem and/or progenitor cell into a fibroblast may be performed by the following procedures. A tissue stem and/or progenitor cell cultured in a serum-free medium is harvested by centrifugation and dispersed by treatment with an enzyme (Accumax^{™}, Innovative Cell Technologies, Inc.) at 37°C. The dispersed cell is cultured in a medium containing 10% serum to thereby induce differentiation into a fibroblast. This step of induction into a fibroblast is an optional step. Among tissue stem and progenitor cells, those which inherently have the nature of fibroblast (such as bone marrow mesenchymal stem cell) do not need special differentiation induction and may be subjected to growth ability decreasing treatment directly.

The fibroblast induced from a tissue stem and/or progenitor cell is treated with radiation, mitomycin or the like to decrease its growth ability. For example, the fibroblast which has reached semi-confluence is cultured in mitomycin C-added medium at 37°C for 2 hours. Alternatively, the fibroblast may be given a lethal dose of radiation.

By co-culturing a cell with the feeder cell of the present invention, it is possible to regulate the growth or differentiation of the resultant cultured cell.

Preferably, the cell to be co-cultured with the feeder cell of the present invention is a human cell.

The type of the cell to be co-cultured with the feeder cell of the present invention is not particularly limited. Examples of cells which may be co-cultured with the feeder cell of the present invention include, but are not limited to, corneal cells (e.g., corneal epithelial cell), skin cells (e.g., epidermal cell), cardiac cells (e.g., cardiomyocyte), gastrointestinal cells (e.g., gastric mucosal epithelial cell, small intestinal mucosal epithelial cell and colonic mucosal epithelial cell), bone marrow cells, embryonic stem cells and oral mucosal cells. When stem and/or progenitor cells are present in the cell to be cultured, it is possible not only to maintain the survival of the cell but also to grow or differentiate the cell by co-culturing with the feeder cell of the present invention. When the cell to be cultured is a tissue cell, the tissue may be regenerated in some occasions. The thus regenerated tissue may be transplanted.

When the cell to be co-cultured with the feeder cell of the present invention is corneal epithelial cell or skin cell, the resultant cultured cell may be grown and stratified.

When the cell to be co-cultured with the feeder cell of the present invention is bone marrow cell or embryonic stem cell, the resultant cultured cell may be grown but would not be stratified.

Co-culture with the feeder cell of the present invention may be performed as described below. First, the feeder cell is seeded in culture dishes to give 70-90% confluence. On the next day, the cell to be co-cultured is seeded. The cell may be cultured in contact with the feeder cell. Alternatively, the cell may be cultured without contact with the feeder cell by using culture inserts or the like.

When a cell such as corneal epithelial cell, skin epidermal cell or oral mucosal epithelial cell is to be stratified, the cell may be cultured until it reaches confluence on culture inserts in a serum-added medium. Subsequently, the medium may be reduced until the cell comes in contact with the liquid-air interface. Then, the cell may be cultured for about one week.

By co-culturing a cell with the feeder cell of the present invention, it is possible to prepare a cultured cell for use in transplantation. In order to coat a wide area with a limited amount of tissue, a graft which is an epithelium cultured on an amniotic membrane is prepared in advance by the method as described above. Briefly, one layer of amniotic membrane is placed in a culture plate, and a cornea from a donor or a small amount of cells collected from a patient is cultured thereon. Transplantation of cultured epithelial sheets is advantageous compared to transplantation of amniotic membrane alone, because the former enables wound healing from an early stage and is effective in inhibiting inflammation. Further, it is suggested that undifferentiated cells are also contained in grown cells. Thus, there is a latent possibility that corneal epithelium may be regenerated from a small amount of stem cells. In surgical operations, abnormal epithelium covering the cornea is completely excised, followed by stanching. Subsequently, a cultured cell sheet is developed over the eye surface and fixed with a surgical suture. A protective contact lens is applied to the eye so that the cultured cell sheet does not fall off. Thus, the surgery is achieved.

Further, the present invention provides a cell culturing kit comprising the above-described feeder cell.

The kit of the present invention may comprise, in addition to the feeder cell, a medium (e.g., DMEM/F12 or DMEM), growth factors (e.g., EGF, insulin, cholera toxin, hydrocortisone, transferrin, isoproterenol , triiodothyronine and adenine) and the like. With the kit of the present invention, it is possible to culture a cell, and the resultant cultured cell may be used for transplantation.

### EXAMPLES

Hereinbelow, the present invention will be described more specifically with reference to the following Examples. However, the scope of the present invention is not limited by these Examples.

### [Example 1]

### Experimental Methods and Materials

Mouse corneal stroma progenitor cells treated with trypsin, collagenase and hyaluronidase were dispersed mechanically, suspended in DMEM/F12 supplemented with 10% fetal bovine serum (FBS), seeded in 75 cm² flasks so that 3x10⁶ cells are present per flask and cultured at 37°C for 4 days to thereby induce fibroblasts according to the method described in Inventigative Ophthalmology & Visual Science, May 2005, Vo1.46, No.5, pp.1653-1658. The cells after induction were treated with 4 µg/ml mitomycin C (Sigma) at 37°C for 2 hours. Subsequently, the cells were dispersed by 0.05% trypsin EDTA (Invitrogen) treatment, diluted with a cell banker (Juzen Field) to give a density of 3x10⁶ cells/ml and cryopreserved with a deep freezer. One day before the seeding of epithelial cells, these cells were thawed, diluted with DMEM/F12 supplemented with 10% fetal bovine serum (FBS), and seeded in 6-well plates (Falcon) at a density of 2.5x10⁴/cm².

On the next day, the limbus was separated from the remaining portion of sclerocorneal sections (from U.S. eye bank) used in corneal transplantation, followed by surgical removal of the iris, ciliary body, Descemet's membrane, endothelium and conjunctiva. The thus prepared sections were treated overnight in a serum-added medium supplemented with Dispase (Invitrogen, 10 mg/ml) and D-sorbitol (Wako, 9 mg/ml) at 4 °C. Subsequently, the epithelium was peeled off from the stroma and then epithelial cells were dispersed by treatment with 0.05% trypsin EDTA at 37°C for 30 minutes. The epithelial cells were seeded in fibrin gel (Bolheal^{™}; KAKETSUKEN; 2 mg/cm²)-coated culture inserts (Corning; Transwell^{™}, cat no 3450) and co-cultured with the feeder cell prepared on the previous day for about two weeks. As a medium, DMEM/F12 supplemented with FBS (10%), human recombinant epidermal growth factor (Invitrogen, 10 ng/ml), human recombinant insulin (Wako, 5 µg/ml), transferrin human cell culture tested (Sigma, 5 µg/ml), hydrocortisone water soluble (Sigma, 0.5 µg/ml), DL-isoproterenol hydrochloride (Sigma 0.25 µg/ml) and streptomycin/penicillin [i.e., Supplement hormonal epithelial medium (SHEM)] to which aprotinin (Wako, 666 KIU/ml) was further added was used and exchanged three times per week. When cells reached confluence, the medium was reduced to expose cells to air, followed by culturing for another 6 days with daily medium exchange.

The thus cultured cells were fixed in formalin, followed by preparation of paraffin sections and HE staining. Alternatively, the cultured cells were embedded in 4% CMC solution (Sakura Finetek) and frozen with liquid nitrogen, followed by preparation of cryosections. The cryosections were fixed in acetone, blocked with PBS consisting of 10 % serum, stained with mouse monoclonal anti-keratin-3 antibody (AE5; Progen Biotechnik GmbH), anti-connexin 43 antibody (Chemicon International Inc.), anti-integrin β1 antibody (Chemicon) and anti-p63 antibody (Oncogene Research Products) and reacted with Cy3-labeled anti-mouse antibody. Then, staining was detected with a fluorescence microscope.

In the measurement of colony forming efficiency, feeder cells were seeded in 100 mm dishes. On the next day, 1000 epithelial cells were seeded on the dishes. After a 2-week culture, cells were fixed with 10% formalin and stained with rhodamin B.

### Results

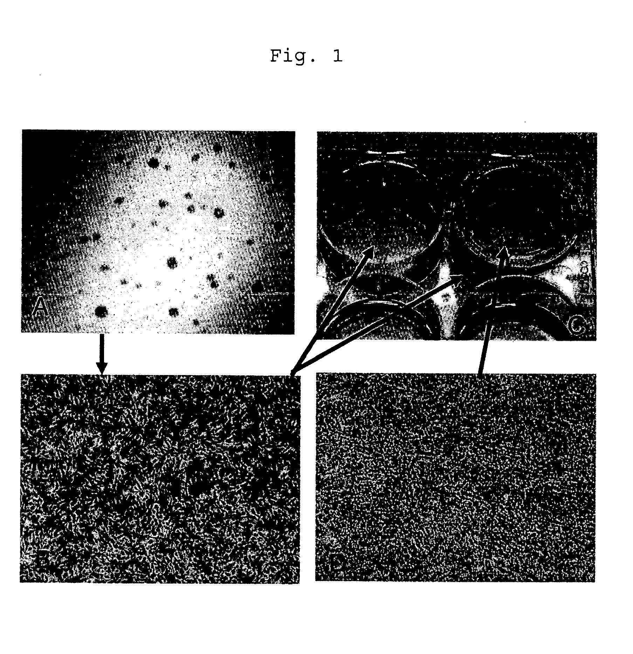

The results are shown in Fig. 1. Fig. 1A shows mouse stromal stem cells in culture. Fig. 1B shows feeder cells obtained from the cells shown in Fig. 1A by treatment with drugs. Fig. 1C shows practice of epithelial cell culture. Feeder cells are placed at the bottoms of culture dishes. Epithelial cells are cultured in culture inserts placed on the feeder cells. Fig. 1D shows epithelial cells in culture.

The cultured epithelial sheet prepared using this feeder cell (shown in the central column in Fig. 2) is stratified as in the cultured epithelial sheet prepared using conventional 3T3 (shown in the left column in Fig. 2). Further, the former epithelial sheet and the latter epithelial sheet show almost similar gene expression patterns in differentiation markers and undifferentiation markers. That is, K3 positive cells were recognized in the outer most layer; Cx43 positive cells were recognized in allover the epithelium including basal cells; and the expression of integrin β1 and p63 was recognized in allover the epithelium except for the outer most layer. These results suggest that the cultured epithelial sheets do not differ in quality between when a feeder cell derived from mouse corneal stroma stem cell is used and when 3T3 is used.

Fig. 3 shows difference in colony forming efficiency when an extremely small number of epithelial cells were seeded. While no colony formation of epithelial cells was recognized without feeder cells, colony formation of epithelial cells was recognized when this feeder cell was used. These results suggest that this feeder cell is able to provide a microenvironment capable of supporting the survival and growth of epithelial cells.

### [Example 2]

### Experimental Methods and Materials

Corneal stroma stem cells (COPs) were cultured and induced into fibroblasts using a serum-added medium according to the method of Yoshida et al. (Invest Ophthalmol Vis Sci. 2005 May; 46(5): 1653-8). Human bone marrow mesenchymal stem cells (MASCs) were supplied by SanBio and cultured according to the SanBio's protocol. When cells reached confluence, they were treated with mitomycin C (4 µg/ml, 37°C, 2 hours) to prepare feeder cells, which were then cryopreserved at -80°C until use.

In order to examine the epithelial growth supporting ability of feeder cells, human limbal epithelial cells were prepared from U.S. eye bank corneas by enzyme treatment, seeded on 100 mm culture dishes (1000 cells/dish) which contain COP or MASC feeder cell, and co-cultured for 2 weeks. As a medium, SHEM (the same medium as used in Example 1) was used. After culturing, the dishes were fixed with formalin and epithelial colonies were stained with rhodamin B. The percentage obtained by dividing the number of colonies by the number of cells seeded was taken as colony forming efficiency.

In order to examine whether or not feeder cells can support the differentiation and stratification of epithelial cells, each feeder cell was placed in 6-well plates and fibrin-coated cell culture inserts. In these inserts, human limbal epithelial cells were three-dimensionally cultured. As in Example 1, Bolheal (KAKETSUKEN; 2 mg/cm²)-coated culture inserts (Corning; Transwell®, cat no 3450) were used. When human epithelial cells reached confluence, a 1-week air lift culture was performed thereafter to thereby prepare cultured epithelial sheets. The resultant epithelial sheets were fixed in formalin, and paraffin sections were prepared therefrom, stained with HE and subjected to histological assay. Alternatively, the epithelial sheets were subjected to immunohistological assay in the form of fresh cryosections. In the immunohistological assay, anti-keratin-3 antibody, anti-keratin-12 antibody, anti-keratin-15 antibody, anti-connexin 43 antibody, anti-integrin β1 antibody or anti-p63 antibody was used as a primary antibody; Alexa flour-labeled antibody (Invitrogen) was used as a secondary antibody; and DAPI was used for nuclear staining. Then, the presence or absence of expression was examined with a fluorescence microscope (Axioplan 2; Carl Zeiss).

### Results

As shown in Fig. 2, cultured epithelial sheets could be prepared by co-culturing with corneal stroma stem cells. These sheets were equivalent to those sheets obtained by co-culturing with conventional mouse 3T3. Specifically, cultured epithelial sheets co-cultured with corneal stroma stem cells were stratified, contained cells expressing differentiation markers K3 and Cx43, and yet contained cells expressing undifferentiation markers Int b1 and p63. Further, as shown in Fig. 3, cultured epithelial sheets co-cultured with corneal stroma stem cells were maintaining cells which retained such growth ability that enabled colony formation. On the other hand, epithelial cells co-cultured with no feeder cell were hardly stratified, showed little expression of differentiation markers, and did not maintain those cells with colony forming ability. As shown in Figs. 4 and 5, epithelial cells co-cultured with a feeder cell derived from human mesenchymal stem cells could also form stratified epithelial sheets. Regardless of whether contacting with the feeder cell or not, these epithelial sheets contained differentiated cells expressing K3 and K12. Especially when humoral factors were continuously supplied (Duplex co-culture and Separated co-culture), expression of K15 was recognized as recognized in the corneal limbus where corneal epithelial stem cells are present.

All publications, patents and patent applications cited herein are incorporated herein by reference in their entirety.

### INDUSTRIAL APPLICABILITY

According to the present invention, a feeder cell is provided. By using the feeder cell of the present invention, it is possible to prepare transplantable cultured cells.

## Claims

1. A feeder cell derived from a tissue stem and/or progenitor cell, wherein the tissue stem and/or progenitor cell is derived from bone marrow mesenchyme.

2. The feeder cell according to claim 1, wherein the tissue stem and/or progenitor cell is derived from a mammal.

3. The feeder cell according to claim 2, wherein the mammal is human.

4. A method of preparing a feeder cell, comprising treating a tissue stem and/or progenitor cell to decrease the growth ability thereof, optionally after inducing the tissue stem and/or progenitor cell into a fibroblast, wherein the tissue stem and/or progenitor cell is derived from bone marrow mesenchyme.

5. The method according to claim 4, wherein the tissue stem and/or progenitor cell is derived from a mammal.

6. The method according to claim 5, wherein the mammal is human.

7. A method of preparing a cultured cell, comprising co-culturing a cell with the feeder cell of any one of claims 1 to 3.

8. The method according to claim 7, wherein the cell co-cultured with the feeder cell of any one of claims 1 to 3 is derived from a mammal.

9. The method according to claim 8, wherein the mammal is human.

10. The method of any one of claims 7 to 9, wherein the cell co-cultured with the feeder cell of any one of claims 1 to 3 is selected from the group consisting of corneal cells, skin cells, gingival cells, cardiac cells, gastrointestinal cells, bone marrow cells, embryonic stem cells and oral mucosal cells.

11. The method according to claim 10, wherein the cell co-cultured with the feeder cell of any one of claims 1 to 3 is a corneal epithelial cell or skin cell.

12. The method according to claim 11, wherein the cultured cell is stratified.

13. The method according to any one of claims 7 to 12, wherein the cultured cell is used for transplantation.

14. A cell culturing kit comprising the feeder cell of any one of claims 1 to 3.
